# EUROPÄISCHE PATENTSCHRIFT

(11) **EP 0 770 083 B1**
(45) Veröffentlichungstag und Bekanntmachung des Hinweises auf die Patenterteilung: **30.08.2000**
(21) Anmeldenummer: 95924949.1
(22) Anmeldetag: 28.06.1995
(51) Int. Cl.: C07D 498/06, A61K 31/535

(54) **PYRIDO[3,2,1-i,j][3,1]BENZOXAZINDERIVATE**
PYRIDO[3,2,1-i,j][3,1]BENZOXAZINE DERIVATIVES
DERIVES DE PYRIDO[3,2,1-i,j][3,1]BENZOXAZINE

(30) Priorität: 11.07.1994 DE 4424369
(43) Veröffentlichungstag der Anmeldung: 02.05.1997
(73) Patentinhaber: BAYER AG, 51368 Leverkusen (DE)
(72) Erfinder: HALLENBACH, Werner, D-40789 Monheim (DE); HIMMLER, Thomas, D-51519 Odenthal (DE); JAETSCH, Thomas, D-50668 Köln (DE); MIELKE, Burkhard, D-51375 Leverkusen (DE); BREMM, Klaus, Dieter, D-45661 Recklinghausen (DE); ENDERMANN, Rainer, D-42113 Wuppertal (DE); PIRRO, Franz, D-40764 Langenfeld (DE); STEGEMANN, Michael, D-51381 Leverkusen (DE); WETZSTEIN, Heinz-Georg, D-51377 Köln (DE)
(86) Internationale Anmeldenummer: EP9502510
(87) Internationale Veröffentlichungsnummer: WO9601829

(56) Entgegenhaltungen:
- EP-A- 0 047 005
- EP-A- 0 234 995
- EP-A- 0 373 531

## Beschreibung

Die Erfindung betrifft neue Pyrido[3,2,1-i,j][3,1]benzoxazinderivate, Verfahren zu ihrer Herstellung sowie diese enthaltende antibakterielle Mittel.

Es ist bereits bekannt geworden, daß Pyridobenzoxazincarbonsäuren antibakteriell wirksam sind. Beispiele hierfür finden sich in EP-O 373 531.

Es wurden nun Verbindungen der allgemeinen Formel (I) gefunden 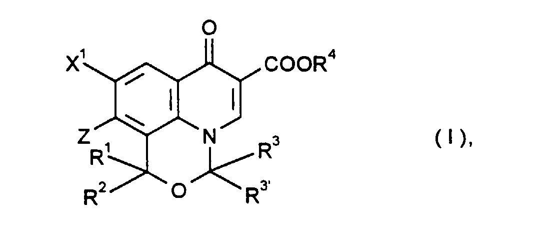 in welcher
- R¹: für Wasserstoff oder gegebenenfalls durch Hydroxy oder Halogen substituiertes C₁-C₄-Alkyl steht,
- R²: unabhängig von R¹ für Wasserstoff oder Methyl steht,
- R³: für Wasserstoff oder C₁-C₄-Alkyl steht,
- R^{3'}: unabhängig von R³ für Wasserstoff oder Methyl steht,
- R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
- X¹: für Wasserstoff oder Halogen steht,
- Z: für Reste der Strukturen 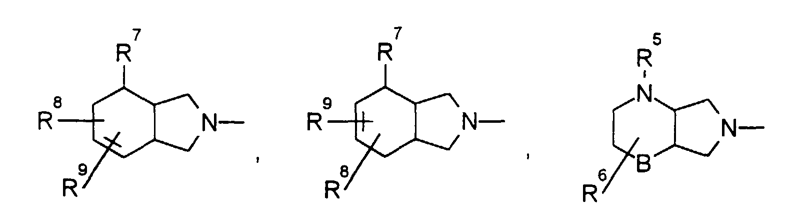 steht,
worin
- R⁷: für Wasserstoff, Hydroxy, -NR¹⁰R¹¹, Hydroxymethyl, -CH₂-NR¹⁰R¹¹, Carboxyl, Methoxycarbonyl oder Ethoxycarbonyl steht,
wobei
R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
R¹¹ für Wasserstoff oder Methyl steht,
- R⁸: für Wasserstoff, geradkettiges oder verzweigtes C₁-C₃-Alkyl oder Cyclopropyl steht,
- R⁹: für Wasserstoff oder Methyl steht,
- R⁶: für Wasserstoff oder Methyl steht,
- R⁵: für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
- R^{5'}: für Methyl oder Ethyl steht,
- B: für -CH₂-, O oder eine direkte Bindung steht.

Die Verbindungen der Formel (I) können in Form von Racematen oder als enantiomerenreine Verbindungen sowie in Form ihrer pharmazeutisch verwendbaren Hydrate und Säureadditionssalze sowie in Form ihrer Alkali-, Erdalkali-, Silber- und Guanidiniumsalze vorliegen.

Man erhält die Verbindungen der Formel (I), wenn man Verbindungen der Formel (II) 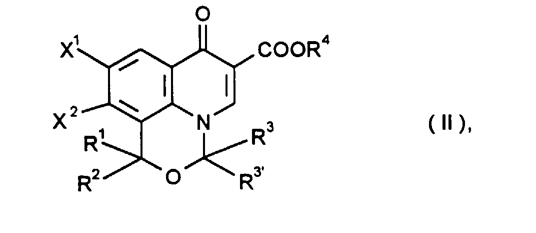 in welcher
R¹, R², R³, R^{3'}, R⁴ und X¹ die oben angegebene Bedeutung haben und
X² für Halogen, insbesondere Fluor oder Chlor, steht,
mit Verbindungen der Formel (III)

Z-H (III),

in welcher
- Z: die oben angegebene Bedeutung hat,
gegebenenfalls in Gegenwart von Säurefängern umsetzt.

Die erfindungsgemäßen Verbindungen weisen im Vergleich zu bekannten Vertretern dieses Strukturtyps eine höhere antibakterielle Wirkung insbesondere im grampositiven Bereich auf. Sie eignen sich daher als Wirkstoffe für die Human- und Veterinärmedizin, wobei zur Veterinärmedizin auch die Behandlung von Fischen zur Therapie oder zur Vorbeugung bakterieller Infektionen zu zählen ist.

Bevorzugt sind Verbindungen der Formel (I), in welcher
- R¹: für Wasserstoff oder gegebenenfalls durch Hydroxy substituiertes C₁-C₃-Alkyl steht,
- R²: unabhängig von R¹ für Wasserstoff oder Methyl steht,
- R³: für Wasserstoff, Methyl oder Ethyl steht,
- R^{3'}: unabhängig von R³ für Wasserstoff oder Methyl steht,
- R⁴: für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
- X¹: für Wasserstoff, Fluor oder Chlor steht,
- Z: für Reste der Strukturen 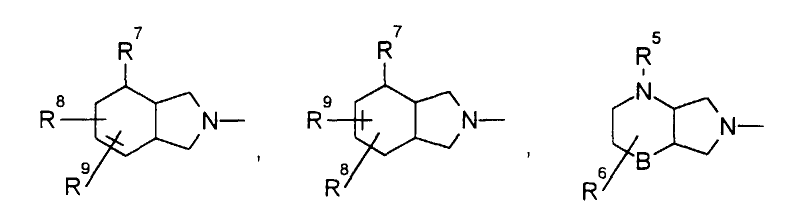 steht,
worin
- R⁷: für Wasserstoff, Hydroxy, -NR¹⁰R¹¹, Hydroxymethyl oder -CH₂-NR¹⁰R¹¹ steht,
wobei
R¹⁰ für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
R¹¹ für Wasserstoff oder Methyl steht,
- R⁸: für Wasserstoff, geradkettiges oder verzweigtes C₁-C₃-Alkyl oder Cyclopropyl steht,
- R⁹: für Wasserstoff oder Methyl steht,
- R⁵: für Wasserstoff oder Methyl steht,
- R⁶: für Wasserstoff steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Besonders bevorzugt sind die Verbindungen der Formel (I), in welcher
- R¹: für Wasserstoff oder Methyl steht,
- R²: für Wasserstoff,
- R³: für Methyl oder Ethyl steht,
- R^{3'}: für Wasserstoff oder Methyl steht,
- R⁴: für Wasserstoff, Methyl oder Ethyl steht,
- X¹: für Fluor steht,
- Z: für Reste der Strukturen 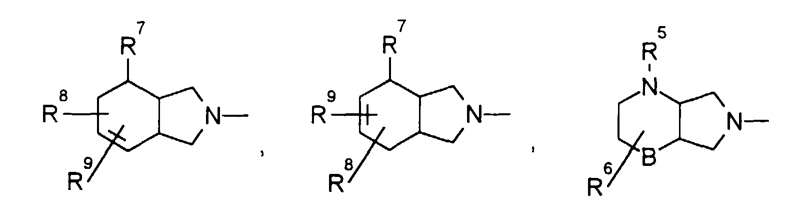 steht,
worin
- R⁷: für Wasserstoff, Hydroxy, -NR¹⁰R¹¹, Hydroxymethyl oder -CH₂-NR¹⁰R¹¹ steht,
wobei
R¹⁰ für Wasserstoff, Methyl, Alkoxycarbonyl mit 1 bis 4 C-Atomen im Alkoxyteil oder C₁-C₃-Acyl steht,
R¹¹ für Wasserstoff oder Methyl steht,
- R⁸: für Wasserstoff, geradkettiges oder verzweigtes C₁-C₃-Alkyl oder Cyclopropyl steht,
- R⁶: für Wasserstoff steht,
- R⁹: für Wasserstoff oder Methyl steht,
- R⁵: für Wasserstoff oder Methyl steht,
- B: für -CH₂-, O oder eine direkte Bindung steht
und deren pharmazeutisch verwendbaren Hydrate und Säureadditionssalze sowie ihre Alkali-, Erdalkali-, Silber- und Guanidiniumsalze.

Im einzelnen seien folgende Verbindungen der Formel (I) genannt:

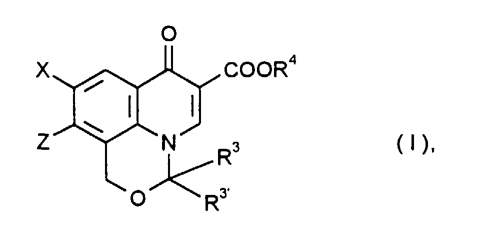

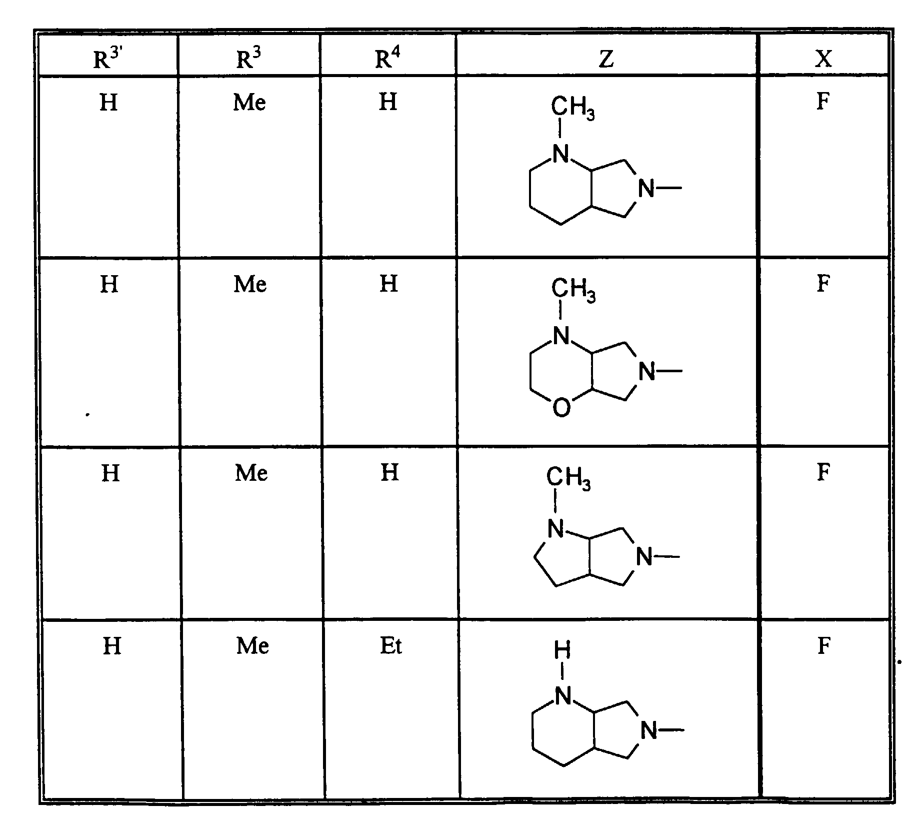

Fortsetzung:

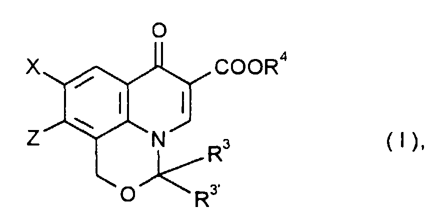

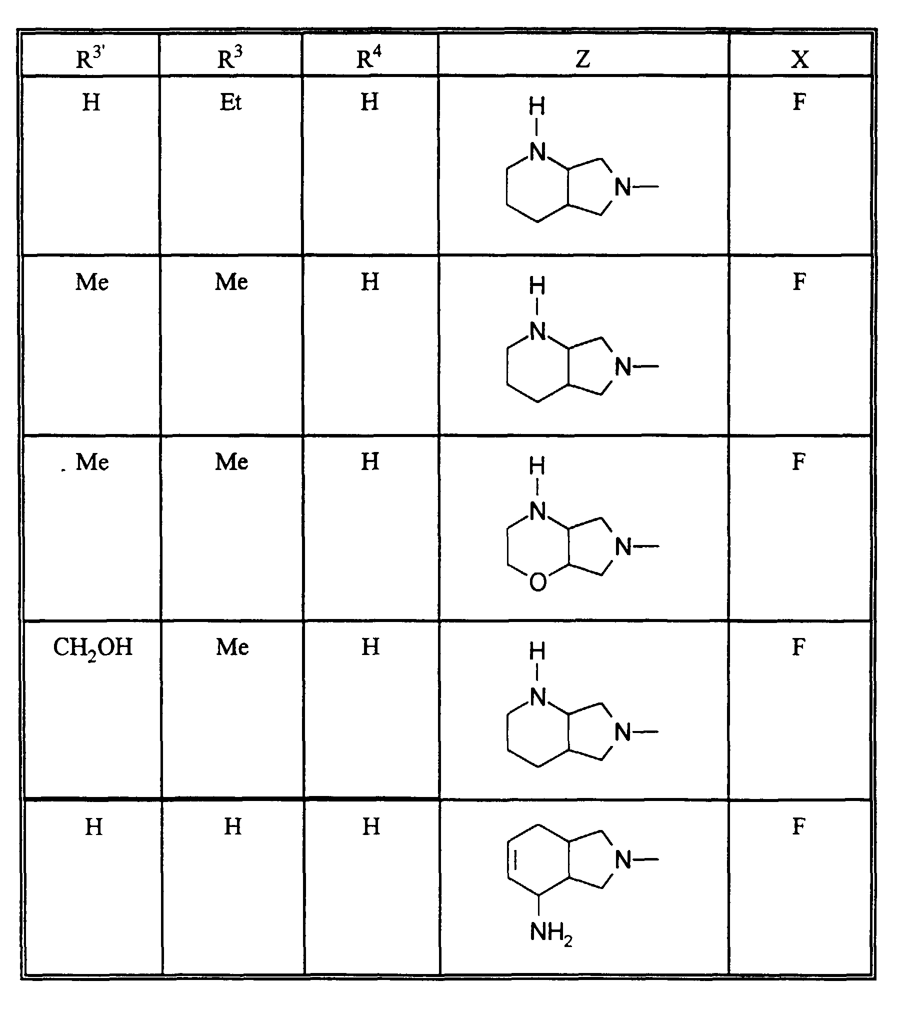

Fortsetzung:

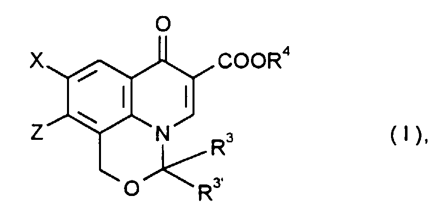

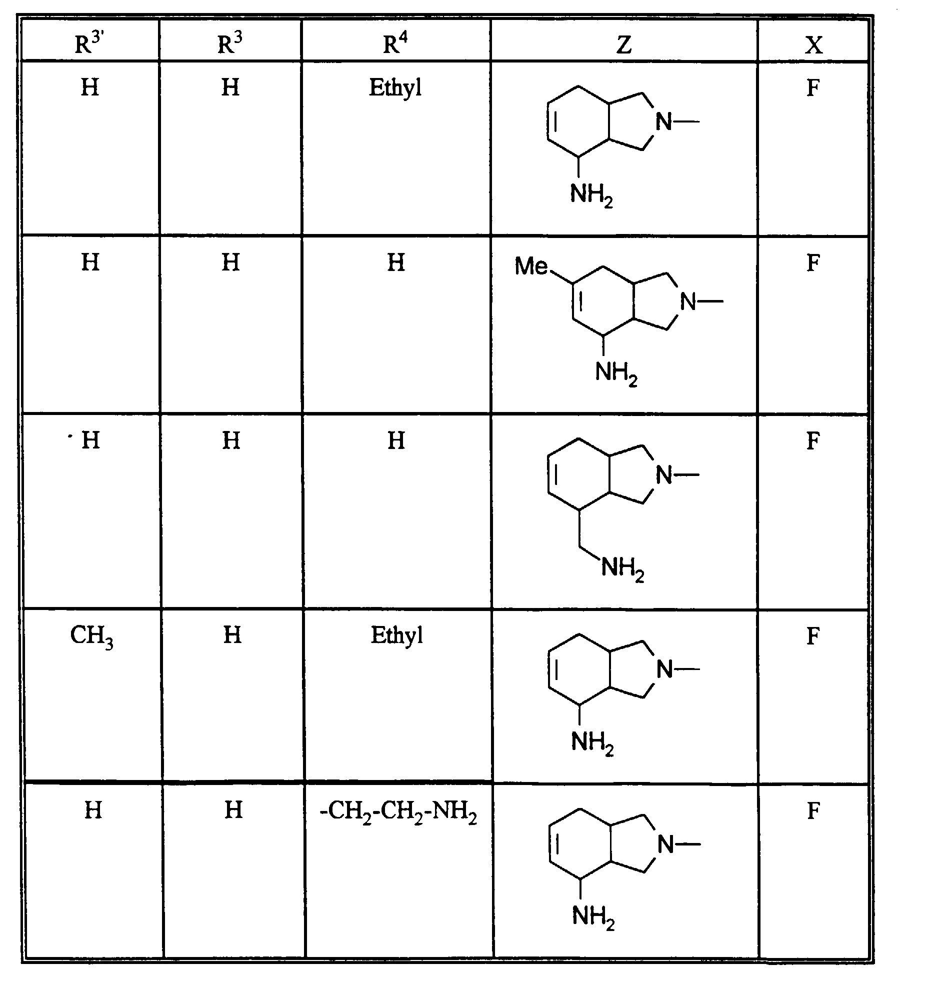

Fortsetzung:

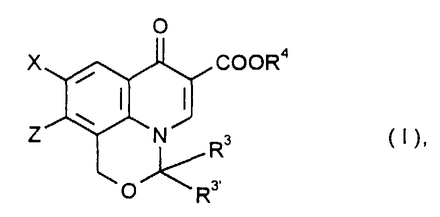

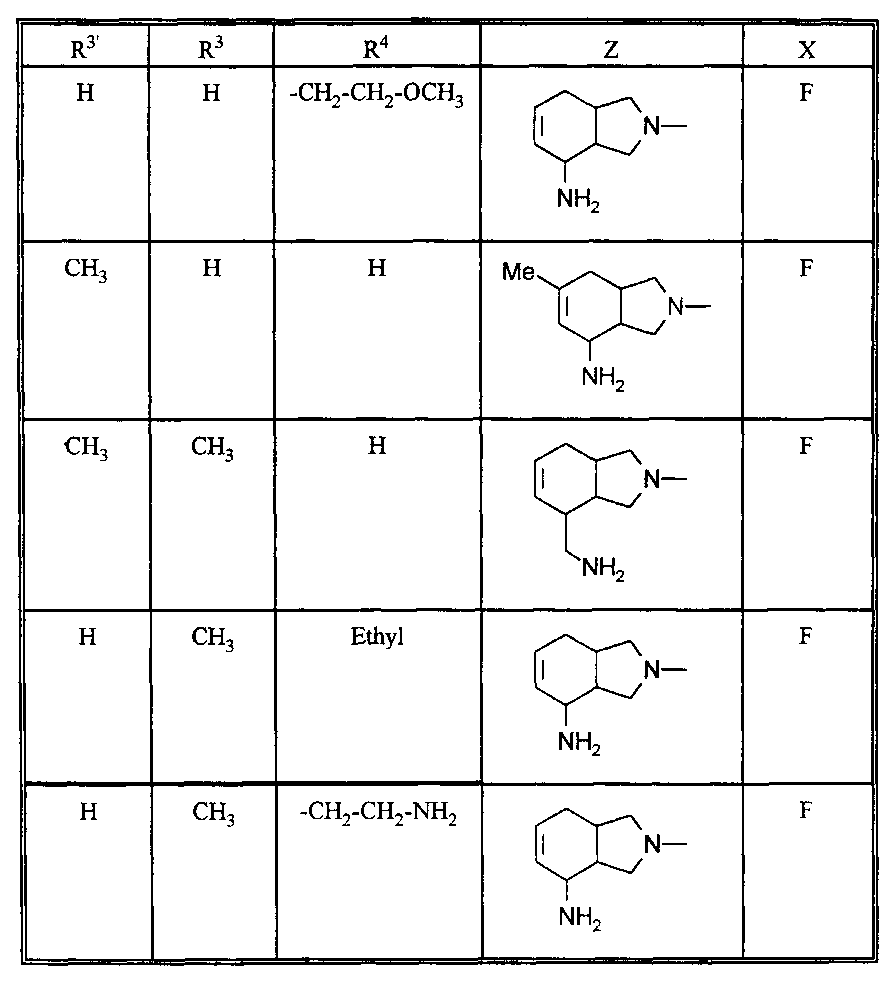

Fortsetzung:

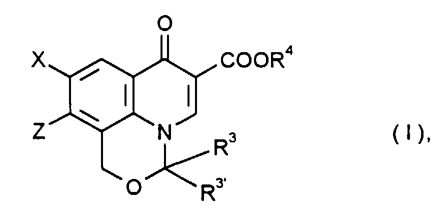

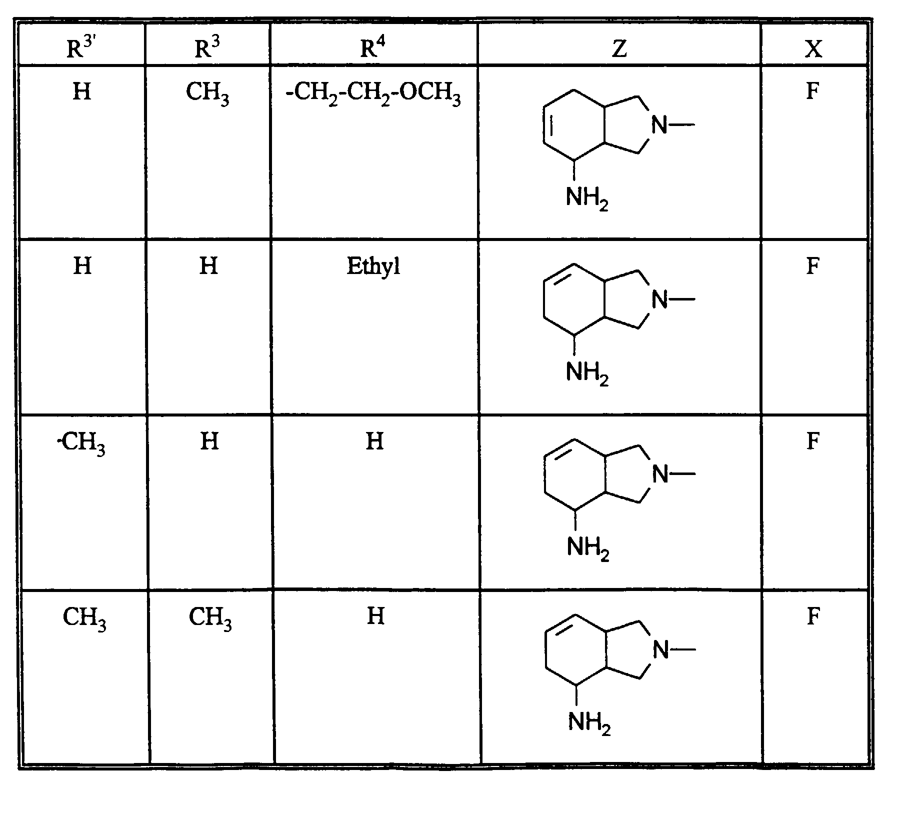

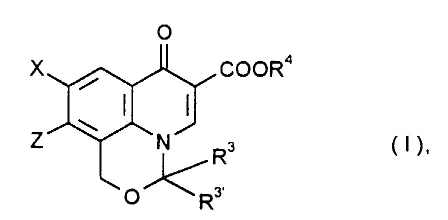

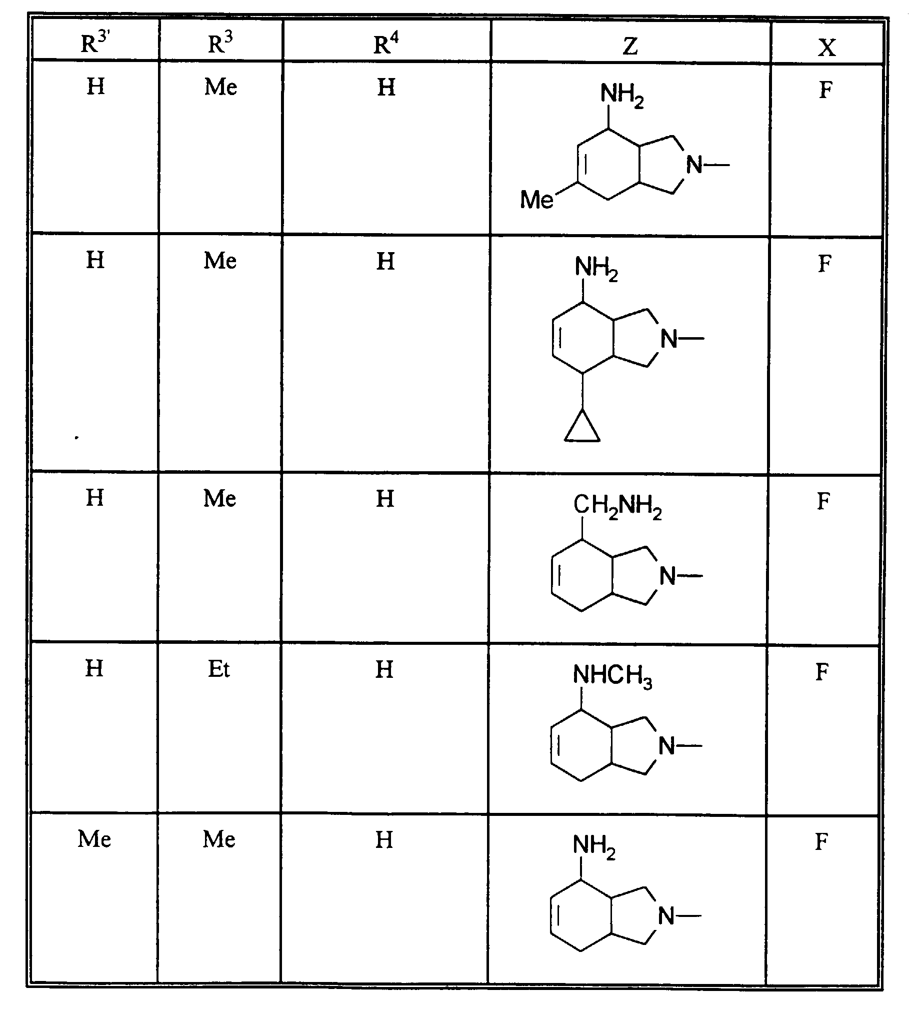

Fortsetzung:

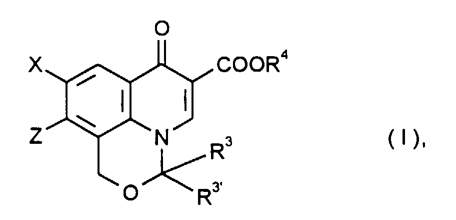

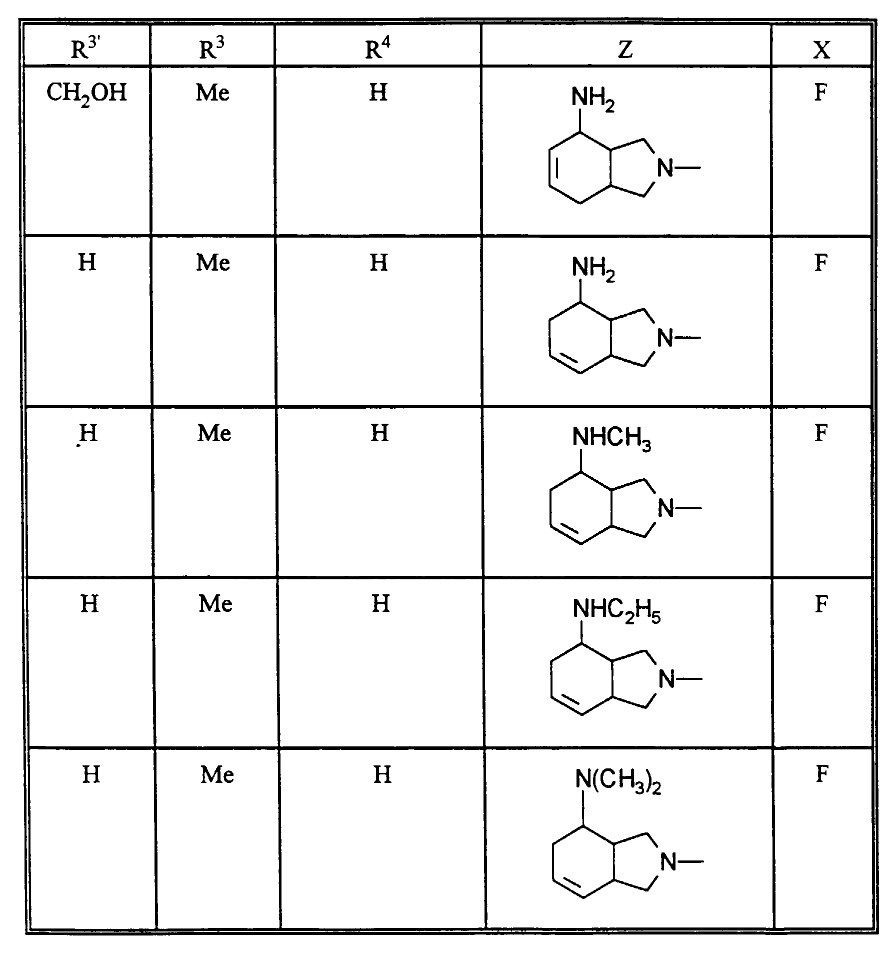

Fortsetzung:

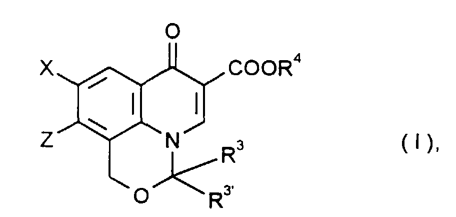

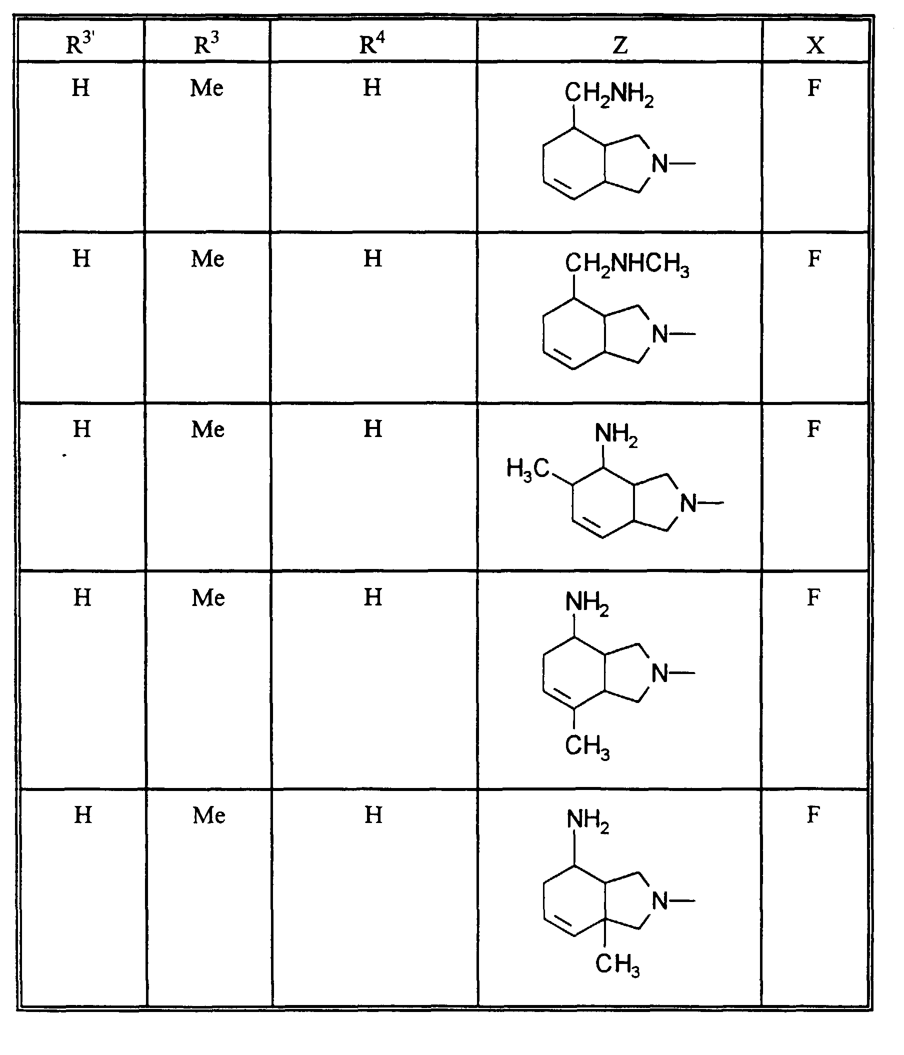

Fortsetzung:

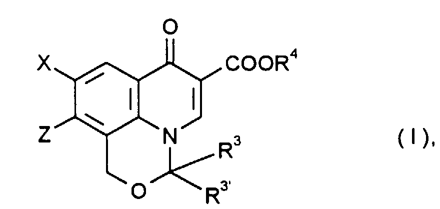

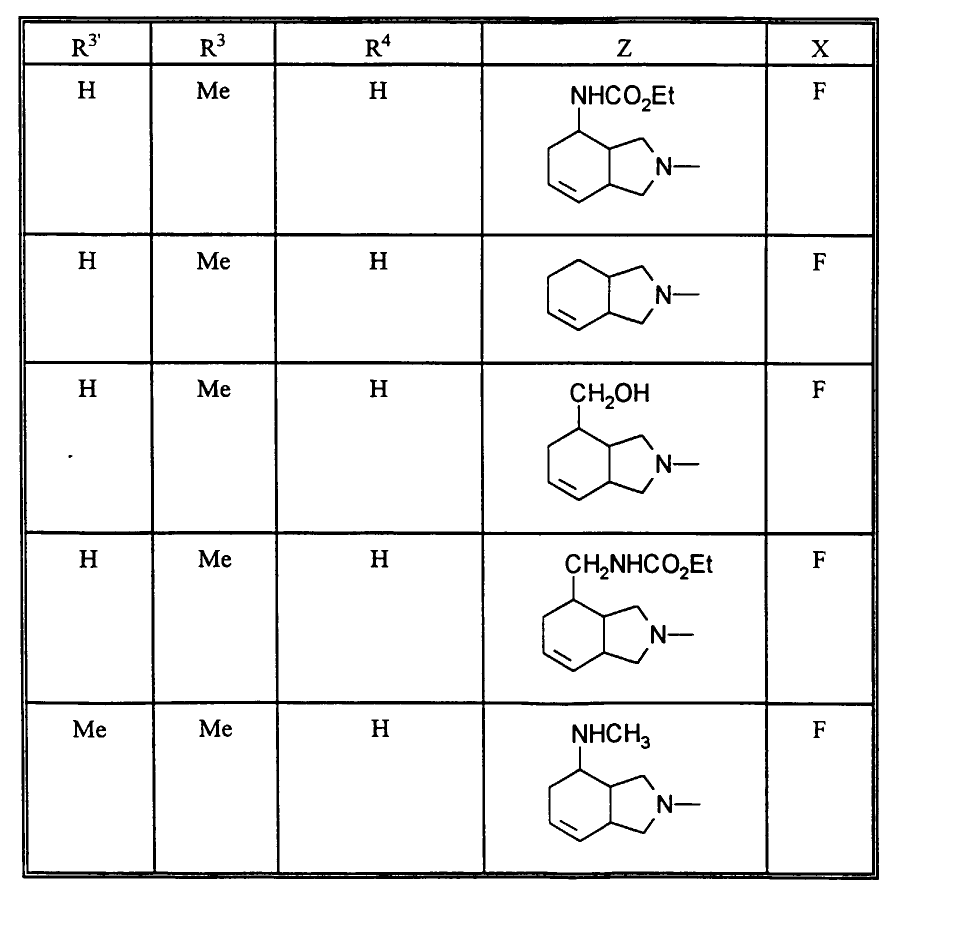

Fortsetzung:

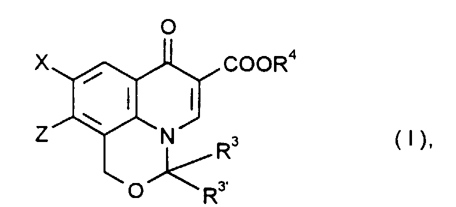

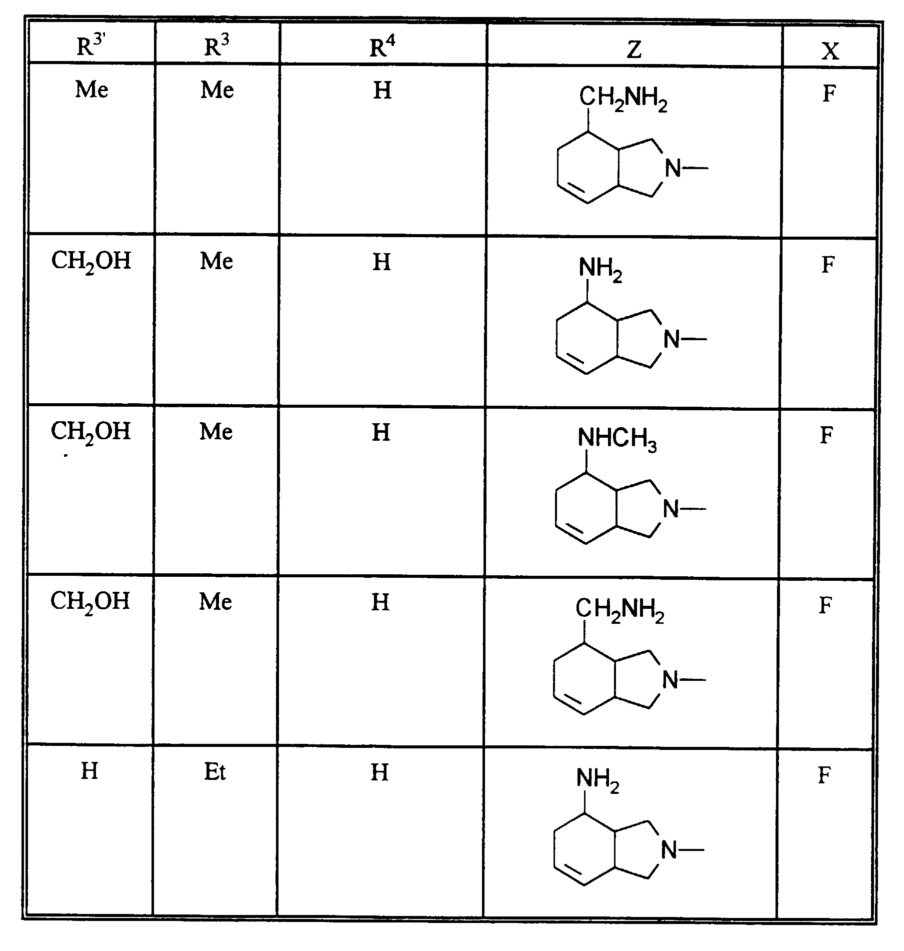

Fortsetzung:

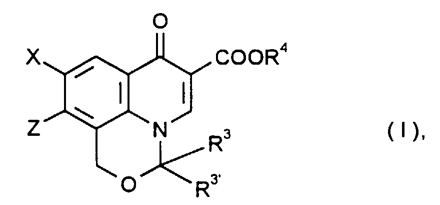

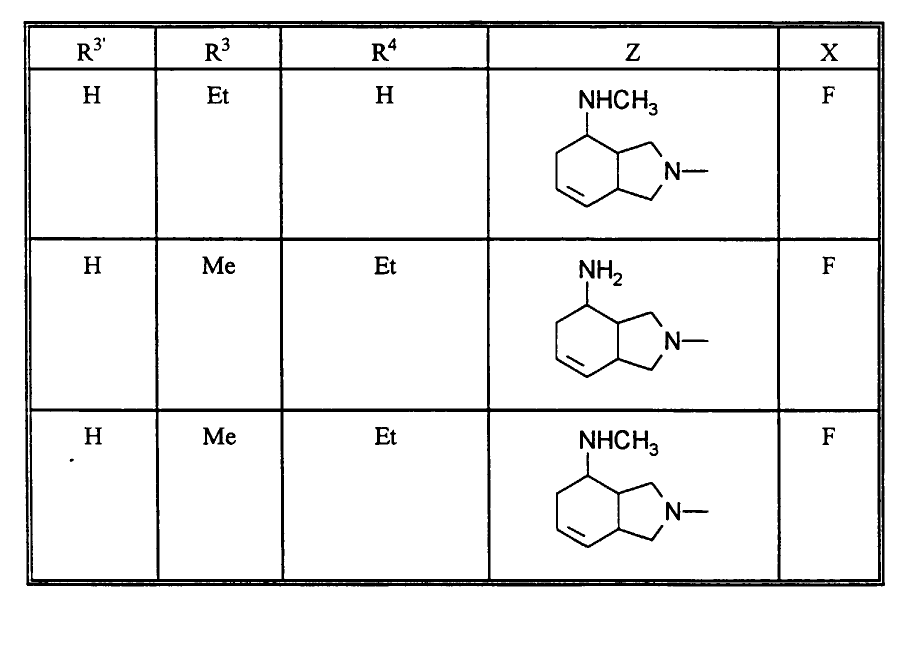

Verwendet man zur Herstellung von Verbindungen der Formel (I) beispielsweise 9,10-Difluor-3-methyl-7-oxo-1H,3H-7H-pyrido[3,2,1-i,j][3,1]benzoxazin-6-carbonsäure und 2,8-Diazabicyclo[4.3.0]nonan, so kann der Reaktionsverlauf durch das folgende Formelschema wiedergegeben werden:

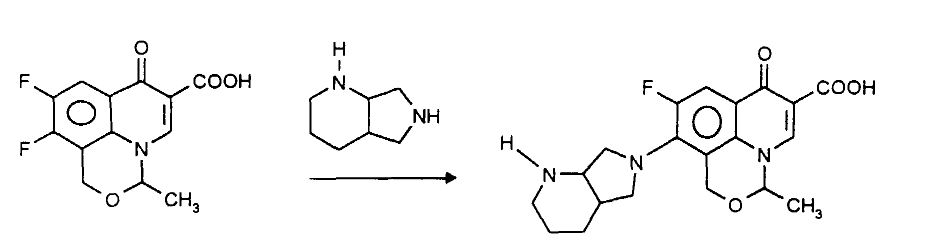

Die als Ausgangsverbindungen verwendeten Verbindungen der Formel (II) sind bekannt, bzw. können nach bekannten Verfahren hergestellt werden. Sie können gegebenenfalls als Racemate, Enantiomere oder reine Diastereomere eingesetzt werden.

Als Beispiele seien genannt:
9,10-Difluor-3-methyl-7-oxo-1H,3H,7H-pyrido-[3,2,1-i,j][3,1]benzoxazin-6-carbonsäure
9,10-Difluor-3-ethyl-7-oxo-1H,3H,7H-pyrido-[3,2,1-i,j][3,1]benzoxazin-6-carbonsäure
9,10-Dichlor-3-methyl-7-oxo-1H,3H,7H-pyrido-[3,2,1-i,j][3,1]benzoxazin-6-carbonsäure
9,10-Difluor-3-dimethyl-7-oxo-1H,3H,7H-pyrido-[3,2,1-i,j][3,1]benzoxazin-6-carbonsäure
9,10-Difluor-3-methyl-7-oxo-1H,3H,7H-pyrido-[3,2,1-i,j][3,1]benzoxazin-6-carbonsäureethylester

Die als Ausgangsverbindungen verwendeten Amine der Formel (III) sind bekannt. Chirale Amine können sowohl als Racemate, als auch als enantiomeren- oder diastereomerenreine Verbindungen eingesetzt werden.

Als Beispiele seien genannt:
2,7-Diazabicyclo[3.3.0]octan
2-Methyl-2,7-diazabicyclo[3.3.0]octan
2,8-Diazabicyclo[4.3.0]nonan
2-Methyl-2,8-diazabicyclo[4.3.0]nonan
2-Oxa-5,8-diazabicyclo[4.3.0]nonan
5-Methyl-2-oxa-5,8-diazabicyclo[4.3.0]nonan.
2-Amino-8-azabicyclo[4.3.0]non-3-en
2-Methylamino-8-azabicyclo[4.3.0]non-3-en
4-Methyl-2-methylamino-8-azabicyclo[4.3.0]non-3-en
5-Methyl-2-methylamino-8-azabicyclo[4.3.0]non-3-en
2-Dimethylamino-8-azabicyclo[4.3.0]non-3-en
2-Ethylamino-8-azabicyclo[4.3.0]non-3-en
2-Methylaminomethyl-8-azabicyclo[4.3.0]non-3-en
2-Hydroxy-8-azabicyclo[4.3.0]non-3-en
5-Isopropyl-2-methylamino-8-azabicyclo[4.3.0]non-3-en
2-Amino-5-isopropyl-8-azabicyclo[4.3.0]non-3-en
2-Amino-5-methyl-8-azabicyclo[4.3.0]non-3-en
2-Hydroxymethyl-8-azabicyclo[4.3.0]non-3-en
2-Amino-5-cyclopropyl-8-azabicyclo[4.3.0]non-3-en
8-Azabicyclo[4.3.0]non-2-en
8-Azabicyclo[4.3.0]non-4-en-2-carbonsäureethylester
2-Hydroxymethyl-8-azabicyclo[4.3.0]non-4-en
2-Amino-8-azabicyclo[4.3.0]non-4-en
2-Ethyloxycarbonylamino-8-azabicyclo[4.3.0]non-4-en
2-tert.-Butyloxycarbonylamino-8-azabicyclo[4.3.0]non-4-en
2-Benzyloxycarbonylamino-8-azabicyclo[4.3.0]non-4-en
2-Allyloxycarbonylaminomethyl-8-azabicyclo[4.3.0]non-4-en
2-Aminomethyl-8-azabicyclo[4.3.0]non-4-en
2-Ethyloxycarbonylaminomethyl-8-azabicyclo[4.3.0]non-4-en
2-tert.-Butyloxycarbonylaminomethyl-8-azabicyclo[4.3.0]non-4-en
2-Methylamino-8-azabicyclo[4.3.0]non-4-en
2-Ethylamino-8-azabicyclo[4.3.0]non-4-en
2-Cyclopropylamino-8-azabicyclo[4.3.0]non-4-en
2-Dimethylamino-8-azabicyclo[4.3.0]non-4-en
2-[(2-Hydroxyethyl)-amino]-8-azabicyclo[4.3.0]non-4-en
2-Amino-1-methyl-8-azabicyclo[4.3.0]non-4-en
2-Amino-2-methyl-8-azabicyclo[4.3.0]non-4-en
2-Amino-3-methyl-8-azabicyclo[4.3.0]non-4-en
2-Ethyloxycarbonylamino-3-methyl-8-azabicyclo[4.3.0]non-4-en
2-tert.-Butyloxycarbonylamino-3-methyl-8-azabicyclo[4.3.0]non-4-en
2-Benzyloxycarbonylamino-3-methyl-8-azabicyclo[4.3.0]non-4-en
2-Allyloxycarbonylaminomethyl-3-methyl-8-azabicyclo[4.3.0]non-4-en
2-Amino-4-methyl-8-azabicyclo[4.3.0]non-4-en
2-Amino-5-methyl-8-azabicyclo[4.3.0]non-4-en
2-Amino-6-methyl-8-azabicyclo[4.3.0]non-4-en
2-Amino-7-methyl-8-azabicyclo[4.3.0]non-4-en
2-Amino-9-methyl-8-azabicyclo[4.3.0]non-4-en

Die substituierten 8-Azabicyclo[4.3.0]non-4-ene und 8-Azabicyclo[4.3.0]non-2-ene sowie ihre Herstellung sind bekannt aus DE-OS 4 230 804.

Sie werden erhalten, indem man geeignete Diene mit geeigneten Dienophilen in einer Diels-Alder Reaktion umsetzt, die intermolekular oder intramolekular durchgeführt werden kann, und gegebenenfalls anschließend weitere chemische Reaktionen durchführt, um gegebenenfalls den Pyrrolidinring aufzubauen und um für die biologische Wirkung gewünschte Substituenten einzuführen und als letzten Schritt die Schutzgruppe am Pyrrolidinstickstoff abspaltet.

Bei intramolekularer Durchführung der Diels-Alder-Reaktion werden Verbindungen der Formel (1) oder (2) 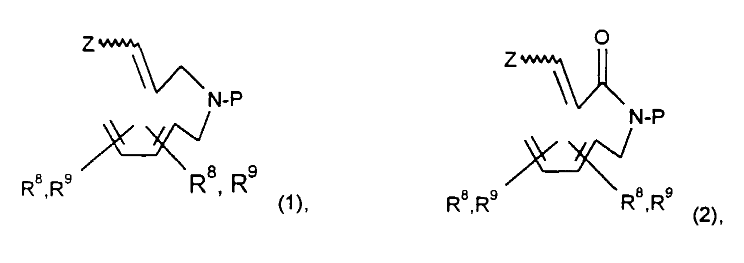 in denen
- R⁸ und R⁹: die oben angegebene Bedeutung haben und
- P: für eine Schutzgruppe (beispielsweise Allyl, Acyl, Carbamoyl oder Trityl),
- Z: für Wasserstoff, eine Carboxyl-, Carbonester- oder Carbonamidgruppe, CN oder NO₂ steht,
zu Verbindungen der Formel (3) [ausgehend von (1)] oder (4) [ausgehend von (2)] 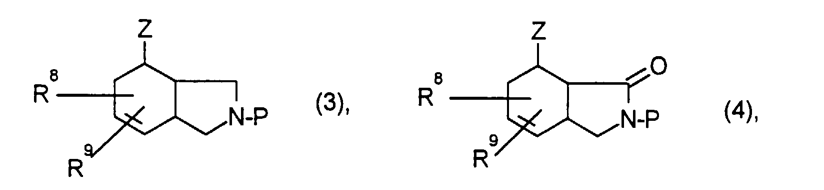 in denen
- R⁸, R⁹, P und Z: die oben angegebenen Bedeutungen haben,
umgesetzt.

Intramolekulare Diels-Alder-Reaktionen ähnlicher Art sind teilweise bekannt: J.M. Mellor, A.M. Wagland; J. Chem. Soc. Perkin I, 997-1005 (1989); W.R. Roush, S.E. Hall; J. Am. Chem. Soc. 103, 5200 (1980); E. Ciganek; Organic Reactions 32, 1-374 (1984). In diesen Arbeiten fehlen jedoch Hinweise auf Schutzgruppen, die gleichzeitig für die Reaktion geeignet und anschließend problemlos abspaltbar sind.

Bei intermolekularer Durchführung der Diels-Alder-Reaktion werden Diene der Formel (5) mit Dienophilen der Formel (6) zu Verbindungen der Formel (7) umgesetzt, und gegebenenfalls nach Modifizierung der Gruppen Z¹ und Z², beispielsweise Überführung eines cyclischen Carbonsäureanhydrids in einen Diester unter Abspaltung der Schutzgruppen P¹ oder P¹ und P², unter Cyclisierung zu den Lactamen der Formel (8) umgesetzt.

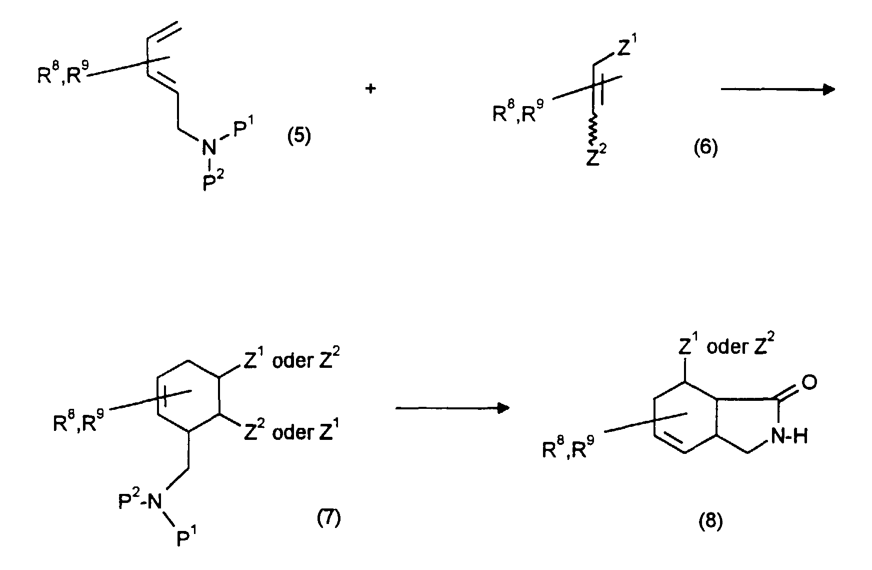

In der Formel (5), (6), (7) und (8) haben R⁸, R⁹ die oben angegebene Bedeutung,
- P¹: steht für eine Acyl- oder Carbamoylschutzgruppe, wenn
- P²: für Wasserstoff steht, oder
- P¹: bildet gemeinsam mit P² ein Imid,
- Z¹ und Z²: stehen für Wasserstoff, Carboxyl, Carbonester- oder Carbonamidgruppen, CN oder NO₂, wobei mindestens eine der beiden Gruppen Z¹ oder Z² eine Carbonestergruppe oder eine Carbonamidgruppe oder CN sein muß oder Z¹ und Z² bilden gemeinsam eine Brücke, so daß ein cyclisches Carbonsäureanhydrid gebildet wird.

Bevorzugte Schutzgruppen P, P¹, P² sind solche Schutzgruppen, bei denen unter den Bedingungen, die zu ihrer Abspaltung verwendet werden, die Cyclisierung zum Lactam und gegebenenfalls eine Veresterung einer zweiten, noch freien Carboxylfunktion mit dem als Lösungsmittel verwendeten Alkohol stattfindet, derart, daß alle Reaktionsschritte in einer Eintopfreaktion ausgeführt werden können, und eine unkontrollierte Umwandlung gegebenenfalls diastereomeren- und enantiomerenreiner Ausgangsstoffe in nicht oder schwer trennbare Isomerengemische nicht stattfindet.

Als Beispiele seien genannt:
1. die tert.-Butyloxycarbonylschutzgruppe (Abspaltung mit wäßrigen oder alkoholischen Säuren)
2. die Phthalimidoschutzgruppe (Aminolyse mit primären Aminen in wäßrigen oder wasserfreien Alkoholen als Lösungsmittel)

Die Umsetzung der Verbindungen der Formel (II) mit Verbindungen der Formel (III), bei der die Verbindungen (III) auch in Form ihrer Salze, wie z.B. der Hydrochloride eingesetzt werden können, wird vorzugsweise in einem Verdünnungsmittel wie Dimethylsulfoxid, N,N-Dimethylformamid, N-Methylpyrrolidon, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo[2.2.2]octan (DABCO), 1,8-Diazabicyclo[5.4.0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drücken zwischen 1 bar und 100 bar, vorzugsweise zwischen 1 und 10 bar.

Bei der Durchführung des erfindungsgemäßen Verfahrens setzt man auf 1 Mol der Verbindung (II) 1 bis 15 Mol, vorzugsweise 1 bis 6 Mol der Verbindung (III) ein.

Freie Aminogruppen können während der Umsetzung durch eine geeignete Aminoschutzgruppe, zum Beispiel durch den tert.-Butoxycarbonylrest, geschützt und nach Beendigung der Reaktion durch Behandlung mit einer geeigneten Säure wie Chlorwasserstoffsäure oder Trifluoressigsäure wieder freigesetzt werden (siehe Houben-Weyl, Methoden der Organischen Chemie, Band E4, Seite 144 (1983); J.F.W. Mc Omie, Protective Groups in Organic Chemistry (1973), Seite 43).

Die erfindungsgemäßen Ester werden durch Umsetzung eines Alkalisalzes der zugrundeliegenden Carbonsäure, die gegebenenfalls am N-Atom durch eine Schutzgruppe wie den tert.-Butoxycarbonylrest geschützt sein kann, mit geeigneten Halogenalkylderivaten in einem Lösungsmittel wie Dimethylformamid, Dimethylacetamid, N-Methylpyrrolidon, Dimethylsulfoxid oder Tetramethylharnstoff bei Temperaturen von etwa 0 bis 100°C, vorzugsweise 0 bis 50°C, erhalten.

Die Herstellung der Säureadditionssalze der erfindungsgemäßen Verbindungen erfolgt in üblicher Weise zum Beispiel durch Lösen des Betains in ausreichender Menge wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser oder einem Alkohol wie Glykolmonoethylether erhitzen und anschließend bis zur Trockne eindampfen oder das ausgefallene Salz absaugen. Als pharmazeutisch verwendbare Salze sind beispielsweise die Salze der Salzsäure, Schwefelsäure, Essigsäure, Glykolsäure, Milchsäure, Bernsteinsäure, Zitronensäure, Weinsäure, Methansulfonsäure, 4-Toluolsulfonsäure, Galacturonsäure, Gluconsäue, Embonsäure, Glutaminsäure oder Asparaginsäure zu verstehen. Ferner lassen sich die erfindungsgemäßen Verbindungen an saure oder basische Ionenaustauscher binden.

Die Alkali- oder Erdalkalisalze der erfindungsgemäßen Carbonsäuren werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze erhalten.

Die erfindungsgemäßen Verbindungen wirken stark antibiotisch und zeigen bei geringer Toxizität ein breites antibakterielles Spektrum gegen grampositive und gramnegative Keime, insbesondere auch gegen solche, die resistent sind gegen verschiedene Antibiotika, wie z.B. Penicilline, Cephalosporine, Aminoglykoside, Sulfonamide, Tetracycline.

Diese wertvollen Eigenschaften ermöglichen ihre Verwendung als chemotherapeutische Wirkung in der Medizin und Tiermedizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gramnegative und grampositive Bakterien und bakterienähnliche Mikroorganismen bekämpft sowie die durch diese Erreger hervorgerufenen Erkrankungen verhindert, gebessert und/oder geheilt werden.

Die erfindungsgemäßen Verbindungen zeichnen sich durch verstärkte Wirkung auf ruhende und resistente Keime aus. Bei ruhenden Bakterien, also Bakterien, die kein nachweisbares Wachstum zeigen, wirken die Verbindungen unterhalb von Konzentrationen ähnlicher Substanzen. Dies bezieht sich nicht nur auf die einzusetzende Menge, sondern auch auf die Geschwindigkeit der Abtötung. Solche Ergebnisse konnten bei grampositiven und -negativen Bakterien, insbesondere bei Staphylococcus aureus, Micrococcus luteus und Enterococcus faecalis beobachtet werden.

Auch gegenüber Bakterien, die gegenüber vergleichbaren Substanzen als weniger empfindlich eingestuft werden, insbesondere resistenten Staphylococcus aureus und Enterococcus faecalis zeigen die erfindungsgemäßen Verbindungen überraschende Wirkungssteigerungen.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Die Verbindungen eignen sich ferner zur Bekämpfung von Protozoonosen und Helminthosen.

Die erfindungsgemäßen Verbindungen können in verschiedenen pharmazeutischen Zubereitungen angewendet werden. Als bevorzugte pharmazeutische Zubereitungen seien Tabletten, Dragees, Kapseln, Pillen, Granulate, Suppositorien, Injektions- und orale verabreichbare Lösungen, Suspensionen und Emulsionen, ferner Pasten, Salben, Gele, Cremes, Lotions, Puder und Sprays genannt.

Die minimalen Hemmkonzentrationen (MHK) wurden per Reihenverdünnungsverfahren auf Iso-Sensitest Agar (Oxoid) bestimmt. Für jede Prüfsubstanz wurde eine Reihe von Agarplatten hergestellt, die bei jeweils doppelter Verdünnung abfallende Konzentrationen des Wirkstoffs enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. 10⁴ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

In der nachstehenden Tabelle sind die MHK-Werte einiger der erfindungsgemäßen Verbindungen im Vergleich zu 9-Fluor-3-methyl-10-(4-methyl-1-piperazinyl)-7-oxo-1H,3H,7H-pyrido[3,2,1-i,j]-[3,1]-benzoxazin-6-carbonsäure (EP-O 373 531) als Referenzverbindung aufgeführt.

**Tabelle:**

| **MHK-Werte** | | | | | |
|---|---|---|---|---|---|
| Spezies | Stamm | Beispiel Nr. | | | Referenz |
| | | 1 | 4 | 5 | |
| E. coli | Z 431 Lit 21 Bui | 0.015 4 | 0.06 4 | <0.015 4 | 0.05 0.05 |
| Klebsiella pneumoniae | 2363 Ge | 0.06 | 0.12 | 0.12 | 0.5 |
| Salmonella | 1 Fr | 0.06 | 0.25 | 0.12 | 1 |
| Enterobacter | 0,4 Ge 02-33 | 0.25 | 0.12 | 0.12 | 1 |
| Staphylococcus aureus | Z 2 Lit 3781 Ge | 0.12 0,12 | 0.5 8 | 0.12 0.12 | 16 128 |
| Pseudomonas | BS 698 TGD | 4 | 8 | 16 | 64 |

### Beispiel 1

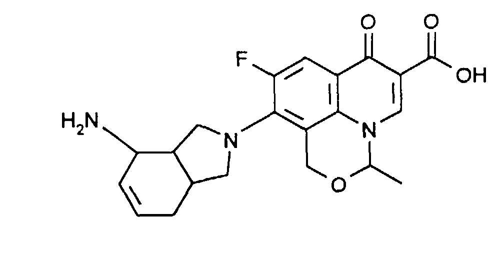

### 10-(2-Amino-8-azabicyclo[4,3,0]non-3-en-8-yl)-9-fluor-3-methyl-7-oxo-1H,3H,7H-pyrido-[3,2,1-i,j][3,1]benzoxazin-6-carbonsäure

293 mg (1,04 mmol) 9,10-Difluor-3-methyl-7-oxo-1H,3H,7H-pyrrido-[3,2,1-i,j][3,1]benzoxazin-6-carbonsäure werden mit 200 mg (1,30 mmol) 2-Amino-8-azabicyclo[4,3,0]non-3-en und 156 mg (1,4 mmol) 1,4-Diazabicyclo[2,2,2]octan in 1,5 ml Dimethylsulfoxid unter Argon 60 Minuten auf 130°C erhitzt. Nach dem Abkühlen wird auf Wasser gegossen, mit verdünnter Salzsäure auf pH 7,5 gestellt und abgesaugt. Es wird mit Wasser nachgewaschen und an der Luft getrocknet. Zur Reinigung wird aus Ethanol umkristallisiert.
Ausbeute: 150 mg (36 % der Theorie)
Schmelzpunkt: 207°C (Zersetzung)
Diastereomerengemisch

Auf analoge Weise werden erhalten:

### Beispiel 2

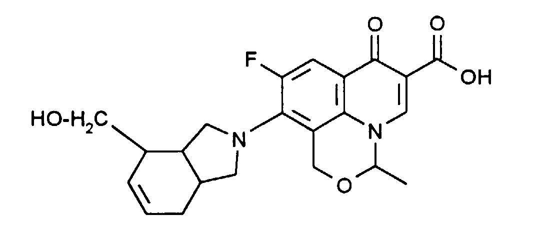

### 10-(2-Hydroxymethyl-8-azabicyclo[4,3,0)non-3-en-8-yl)-9-fluor-3-methyl-7-oxo-1H,3H,7H-pyrido-[3,2,1-i,j][3,1]benzoxazin-6-carbonsäure

Schmelzpunkt: 230°C (Zersetzung)
Diastereomerengemisch

### Beispiel 3

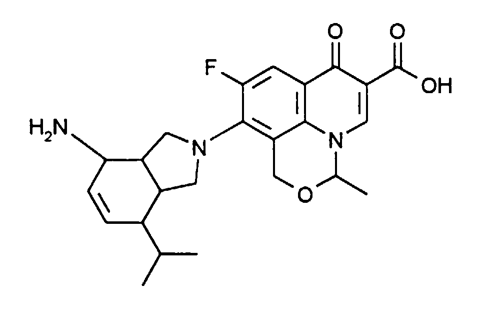

### 10-(2-Amino-5-isopropyl-8-azabicyclo[4,3,0]non-3-en-8-yl)-9-fluor-3-methyl-7-oxo-1H,3H,7H-pyrido-[3,2,1-i,j][3,1]benzoxazin-6-carbonsäure

Schmelzpunkt: 150°C (Zersetzung)
Diastereomerengemisch

### Beispiel 4

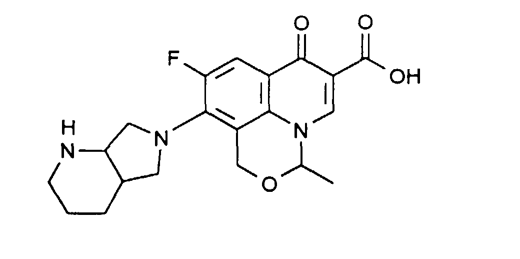

### 10-(2.8-Diazabicyclo[4,3,0]nonan-8-yl)-9-fluor-3-methyl-7-oxo-1H,3H,7H-pyrido-[3,2,1-i,j][3,1]benzoxazin-6-carbonsäure

Schmelzpunkt: 185°C (Zersetzung)
Diastereomerengemisch

### Beispiel 5

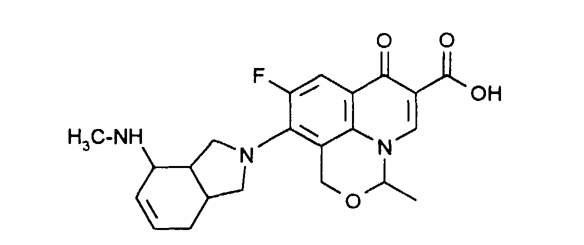

### 9-Fluor-3-methyl-10-(2-methylamino-8-azabicyclo[4.3.0]non-3-en-8-yl)-7-oxo-1H,3H,7H-pyrido[3,2,1-i,j][3,1]benzoxazin-6-carbonsäure

Schmelzpunkt: 200°C (Zersetzung)
Diastereomerengemisch

### Beispiel 6

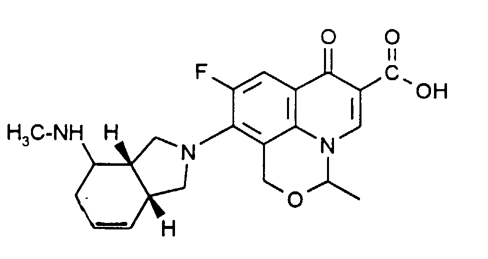

### 9-Fluor-3-methyl-10-(2-methylamino-8-azabicyclo[4.3.0]non-4-en-8-yl)-7-oxo-1H,3H,7H-pyrido[3,2,1-i,j][3,1]benzoxazin-6-carbonsäure

Schmelzpunkt: 290°C (Zersetzung)
Diastereomerengemisch

### Herstellung der Zwischenprodukte:

### Beispiel A:

### 8-Azabicyclo[4.3.0]non-2-en

### A.1. (E)-1-Brom-2,4-pentadien

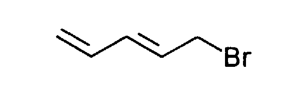

84 g (1,0 mol) 1,4-Pentadien-3-ol bei 0 °C vorlegen. Unter Rühren 150 ml (≈ 1,3 mol) 48 %-ige wäßrige Bromwasserstoffsäure so zutropfen, daß die Innentemperatur 5°C nicht überschreitet. Nach vollständiger Zugabe 1 h bei Raumtemperatur nachrühren. Die organische Phase wird abgetrennt und ohne Reinigung weiter umgesetzt.
Ausbeute: 107-129 g (73-88 % der Theorie)

### A.2. (E)-1-(2-Propenylamino)-2,4-pentadien

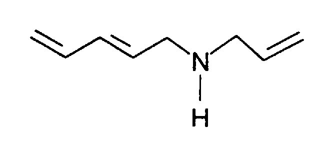

228 g (4,0 mol) 1-Amino-2-propen vorlegen. Unter Rühren 58,8 g (0,4 mol) (E)-1-Brom-2,4-pentadien (Titelverbindung aus Beispiel A.1.) zutropfen. Durch Kühlung die Innentemperatur im Bereich von 20-30°C halten. 5 h bei Raumtemperatur rühren. Ansatz bei 150 mbar einengen. 20 g (0,5 mol) Natriumhydroxid in 200 ml Wasser gelöst zugeben, mit zweimal je 100 ml Methylenchlorid extrahieren, mit Natriumsulfat trocknen, 0,1 g 4-Hydroxyanisol zusetzen, einengen, bei 40 mbar destillieren. Zur Stabilisierung werden dem Destillat 10-20 ppm 4-Hydroxyanisol zugesetzt.
Ausbeute: 33-35 g (67-72 % der Theorie)
Siedepunkt: 77-82 °C bei 40 mbar
¹H-NMR (CDCl₃): δ = 6,07-6,48 (m, 2H); 5,64-6,07 (m, 2H); 5,00-5,27 (m, 4H); 3,19-3,36 ppm (m, 4H).

### A3. N-[(E)-2,4-Pentadienyl]-N-(2-propenyl)-acetamid

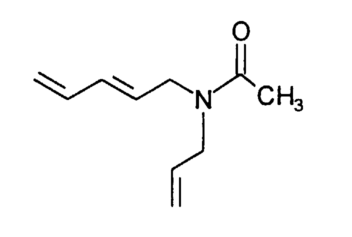

24,6 g (0,2 mol) (E)-1-(2-propenylamino)-2,4-pentadien (Titelverbindung aus Beispiel A.2.) vorlegen, 22,4 g Essigsäureanhydrid zutropfen und über Nacht bei Raumtemperatur rühren. Einengen und als Rohprodukt weiter umsetzen.

### A.4. 8-Acetyl-8-azabicyclo[4.3.0]non-2-en

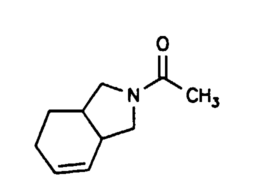

33,1 g (0,2 mol) N-[(E)-2,4-Pentadienyl]-N-(2-propenyl)-acetamid (Titelverbindung aus Beispiel A.3.) in 200 ml Xylol lösen, 15 min einen kräftigen Stickstoffstrom durchleiten, 0,1 g 4-Hydroxyanisol zusetzen, dann über Nacht zum Rückfluß erhitzen. Einengen, im Hochvakuum destillieren.
Ausbeute: 23,1 g (70 % der Theorie bezogen auf die Titelverbindung aus Beispiel A.2.)
Siedepunkt: 88-93 °C bei 0,05 mbar

### A. 5.8-Azabicyclo[4.3.0]non-2-en

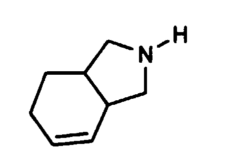

16,5 g (0,1 mol) 8-Acetyl-8-azabicyclo[4.3.0]non-2-en (Titelverbindung aus Beispiel A.4.) in einer Mischung aus 100 ml 45 %-iger Natronlauge, 50 ml Wasser und 100 ml 1,2-Ethandiol 3 h zum Rückfluß erhitzen. Nach Abkühlung viermal mit je 50 ml Diethylether extrahieren. Vereinigte organische Phasen mit Natriumsulfat trocknen, im Hochvakuum destillieren.
Ausbeute: 6,6 g (54 % der Theorie)
Siedepunkt: 36-44 °C bei 0,35 mbar

¹H-NMR (CDCl₃): δ = 5,79 (m, 1H); 5,74 (m, 1H) ; 3,02-3,17 (m, 2H); 2,47-2,72 (m, 2H); 2,06-2,30 (m, 2H); 1,91-2,06 (m, 2H); 1,68 (m, 1H); 1,45 ppm (m, 1H).

## Patentansprüche

1. Verbindungen der allgemeinen Formel (I) 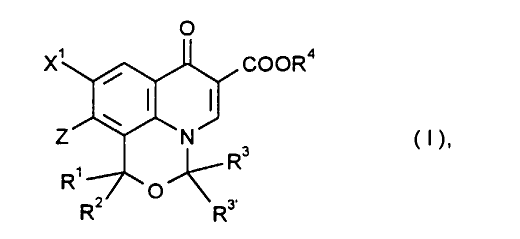 in welcher
R¹ für Wasserstoff oder gegebenenfalls durch Hydroxy oder Halogen substituiertes C₁-C₄-Alkyl steht,
R² unabhängig von R¹ für Wasserstoff oder Methyl steht,
R³ für Wasserstoff oder C₁-C₄-Alkyl steht,
R^{3'} unabhängig von R³ für Wasserstoff oder Methyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)-methyl steht,
X¹ für Wasserstoff oder Halogen steht,
Z für Reste der Strukturen 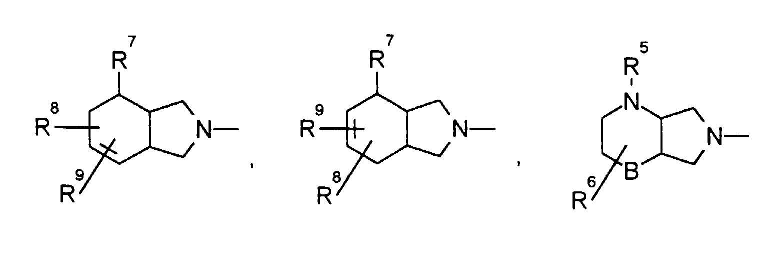 steht,
worin
R⁷ für Wasserstoff, Hydroxy, -NR¹⁰R¹¹, Hydroxymethyl oder -CH₂-NR¹⁰R¹¹, Carboxyl, Methoxycarbonyl oder Ethoxycarbonyl steht,
wobei
R¹⁰ für Wasserstoff, gegebenenfalls durch Hydroxy substituiertes C₁-C₃-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁶ für Wasserstoff oder Methyl steht,
R⁵ für Wasserstoff, Methyl oder Reste der Strukturen -CH=CH-CO₂R^{5'}, -CH₂-CH₂-CO₂R^{5'}, -CH₂-CO-CH₃, -CH₂-CH₂-CN steht,
R^{5'} für Methyl oder Ethyl steht,
B für -CH₂-, O oder eine direkte Bindung steht.
Die Verbindungen der Formel (I) können in Form von Racematen oder als enantiomerenreine Verbindungen sowie Form ihrer pharmazeutisch verwendbaren Hydrate und Säureadditionssalze sowie in Form ihrer Alkali-, Erdalkali-, Silber- und Guanidiniumsalze vorliegen.

2. Verfahren zur Herstellung der Verbindungen der Formel (I) gemäß Anspruch 1 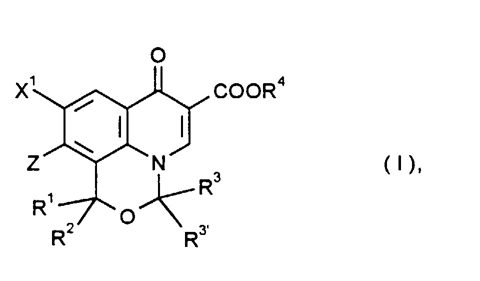 dadurch gekennzeichnet, daß man Verbindungen der Formel (II) 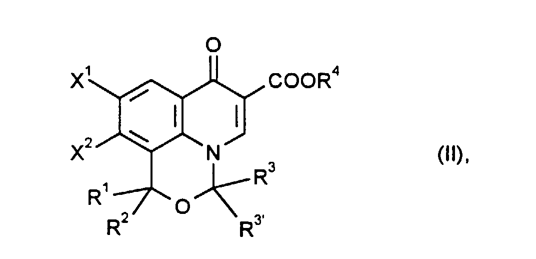 in welcher
R¹, R², R³, R^{3'}, R⁴ und X¹ die oben angegebene Bedeutung haben und
X² für Halogen, insbesondere Fluor oder Chlor, steht,
mit Verbindungen der Formel (III)
Z-H (III),
in welcher
Z die oben angegebene Bedeutung hat,
gegebenenfalls in Gegenwart von Säurefängern umsetzt.

3. Verbindungen der Formel (I) gemäß Anspruch 1, in welcher
R¹ für Wasserstoff oder gegebenenfalls durch Hydroxy substituiertes C₁-C₃-Alkyl steht,
R² unabhängig von R¹ für Wasserstoff oder Methyl steht,
R³ für Wasserstoff, Methyl oder Ethyl steht,
R^{3'} für Wasserstoff oder Methyl steht,
R⁴ für Wasserstoff, gegebenenfalls durch Hydroxy, Methoxy, Amino, Methylamino oder Dimethylamino substituiertes Alkyl mit 1 bis 4 Kohlenstoffatomen oder (5-Methyl-2-oxo-1,3-dioxol-4-yl)methyl steht,
X¹ für Wasserstoff, Fluor oder Chlor steht,
Z für Reste der Strukturen 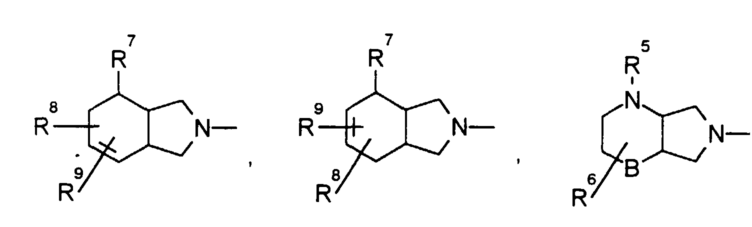 steht,
worin
R⁷ für Wasserstoff, Hydroxy, -NR¹⁰R¹¹, Hydroxymethyl oder -CH₂-NR¹⁰R¹¹ steht,
wobei
R¹⁰ für Wasserstoff, gegebenenfalls durch Hydroxy substituiertes C₁-C₂-Alkyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁹ für Wasserstoff oder Methyl steht,
R⁵ für Wasserstoff oder Methyl steht,
R⁶ für Wasserstoff steht,
B für -CH₂-, O oder eine direkte Bindung steht.

4. Verbindungen der Formel (I) gemäß Anspruch 1, in welcher
R¹ für Wasserstoff oder Methyl steht,
R² für Wasserstoff steht,
R³ für Methyl oder Ethyl steht,
R^{3'} für Wasserstoff oder Methyl steht,
R⁴ für Wasserstoff, Methyl oder Ethyl steht,
X¹ für Fluor steht,
Z für Reste der Strukturen 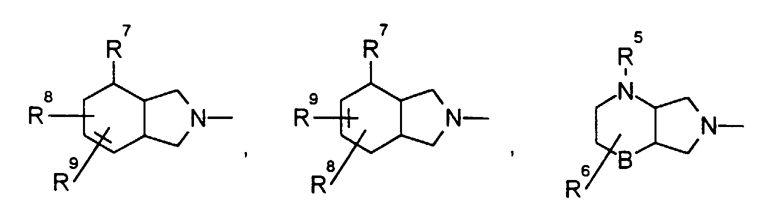 steht,
worin
R² für Wasserstoff, Hydroxy, -NR¹⁰R¹¹, Hydroxymethyl oder -CH₂-NR¹⁰R¹¹ steht,
wobei
R¹⁰ für Wasserstoff, Methyl, Alkoxycarbonyl mit 1 bis 4 C-Atomen im Alkoxyteil oder C₁-C₃-Acyl steht,
R¹¹ für Wasserstoff oder Methyl steht,
R⁸ für Wasserstoff, geradkettiges oder verzweigtes C₁-C₃-Alkyl oder Cyclopropyl steht,
R⁶ für Wasserstoff stehen,
R⁹ für Wasserstoff oder Methyl steht,
R⁵ für Wasserstoff oder Methyl steht,
B für -CH₂-, O oder eine direkte Bindung steht

5. Arzneimittel enthaltend Verbindungen der Formel (I) gemäß Anspruch 1.

6. Verwendung von Verbindungen der Formel (I) gemäß Anspruch 1 zur Herstellung von Arzneimitteln.

7. Verwendung von Verbindungen der Formel (I) gemäß Anspruch 1 in antibakteriellen Mitteln.

## Claims

1. Compounds of the general formula (I) 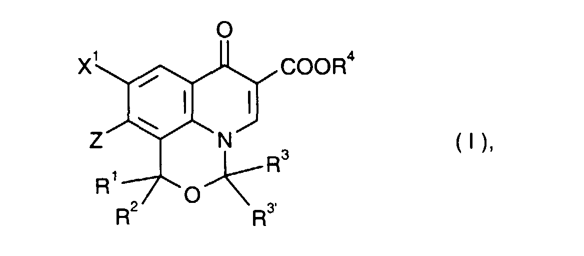 in which
R¹ represents hydrogen or C₁-C₄-alkyl which is optionally substituted by hydroxyl or halogen,
R² independently of R¹ represents hydrogen or methyl,
R³ represents hydrogen or C₁-C₄-alkyl,
R^{3'} independently of R³ represents hydrogen or methyl,
R⁴ represents hydrogen, alkyl having 1 to 4 carbon atoms which is optionally substituted by hydroxyl, methoxy, amino, methylamino or dimethylamino, or represents (5-methyl-2-oxo-1,3-dioxol-4-yl)-methyl,
X¹ represents hydrogen or halogen,
Z represents radicals of the structures 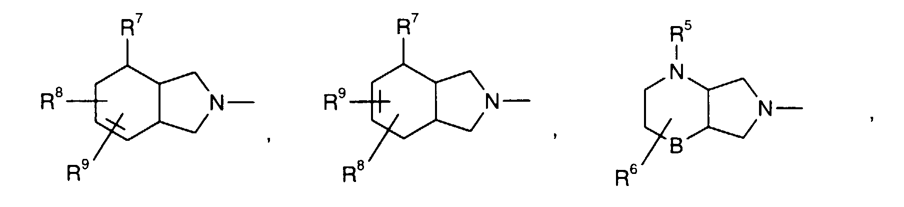 in which
R² represents hydrogen, hydroxyl, -NR¹⁰R¹¹, hydroxymethyl or, -CH₂-NR¹⁰R¹¹, carboxyl, methoxycarbonyl or ethoxycarbonyl,
where
R¹⁰ represents hydrogen, C₁-C₃-alkyl which is optionally substituted by hydroxyl, or represents alkoxycarbonyl having 1 to 4 C atoms in the alkoxy moiety, or C₁-C₃-acyl,
R¹¹ represents hydrogen or methyl,
R⁸ represents hydrogen, straight-chain or branched C₁-C₃-alkyl or cyclopropyl,
R⁹ represents hydrogen or methyl,
R⁶ represents hydrogen or methyl,
R⁵ represents hydrogen, methyl or radicals of the structures -CH=CH-CO₂R^{5'}, -CH₂-CH₂-CO₂R^{5'}, -CH₂-CO-CH₃, -CH₂-CH₂-CN,
R^{5'} represents methyl or ethyl, and
B represents -CH₂-, O or a direct bond.
The compounds of the formula (I) can exist in the form of racemates or enantiomerically pure compounds, in the form of their pharmaceutically utilizable hydrates and acid addition salts, and in the form of their alkali metal salts, alkaline earth metal salts, silver salts and guanidinium salts.

2. Process for the preparation of the compounds of the formula (I) according to Claim 1 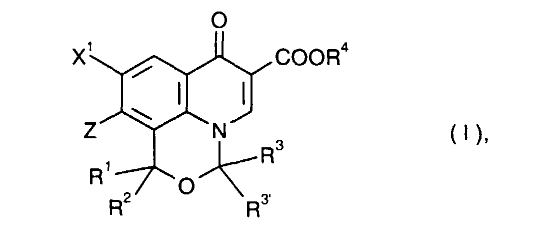 characterized in that compounds of the formula (II) 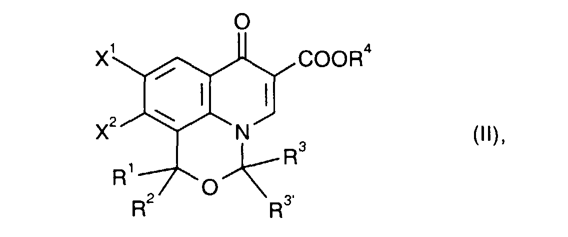 in which
R¹, R², R³, R^{3'}, R⁴ and X¹ have the abovementioned meaning and
X² represents halogen, in particular fluorine or chlorine,
are reacted with compounds of the formula (III)
Z-H (III),
in which
Z has the abovementioned meaning,
if appropriate in the presence of acid scavengers.

3. Compounds of the formula (I) according to Claim 1, in which
R¹ represents hydrogen or C₁-C₃-alkyl which is optionally substituted by hydroxyl,
R² independently of R¹ represents hydrogen or methyl,
R³ represents hydrogen, methyl or ethyl,
R^{3'} represents hydrogen or methyl,
R⁴ represents hydrogen, alkyl having 1 to 4 carbon atoms which is optionally substituted by hydroxyl, methoxy, amino, methylamino or dimethylamino, or represents (5-methyl-2-oxo-1,3-dioxol-4-yl)methyl,
X¹ represents hydrogen, fluorine or chlorine,
Z represents radicals of the structures 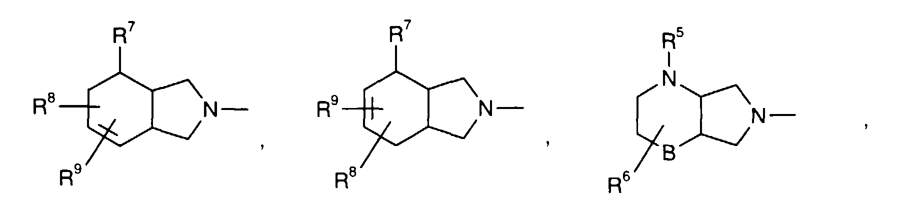 in which
R⁷ represents hydrogen, hydroxyl, -NR¹⁰R¹¹, hydroxymethyl or -CH₂-NR¹⁰R¹¹,
where
R¹⁰ represents hydrogen, C₁-C₂-alkyl which is optionally substituted by hydroxyl, or represents alkoxycarbonyl having 1 to 4 C atoms in the alkoxy moiety, or C₁-C₃-acyl,
R¹¹ represents hydrogen or methyl,
R⁸ represents hydrogen, straight-chain or branched C₁-C₃-alkyl or cyclopropyl,
R⁹ represents hydrogen or methyl,
R⁵ represents hydrogen or methyl,
R⁶ represents hydrogen, and
B represents -CH₂-, O or a direct bond.

4. Compounds of the formula (I) according to Claim 1, in which
R¹ represents hydrogen or methyl,
R² represents hydrogen,
R³ represents methyl or ethyl,
R^{3'} represents hydrogen or methyl,
R⁴ represents hydrogen, methyl or ethyl,
X¹ represents fluorine,
Z represents radicals of the structures 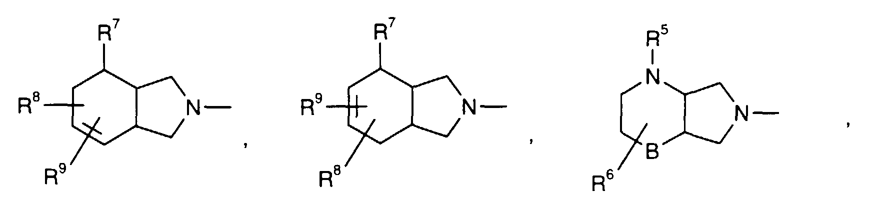 in which
R⁷ represents hydrogen, hydroxyl, -NR¹⁰R¹¹, hydroxymethyl or -CH₂-NR¹⁰R¹¹,
where
R¹⁰ represents hydrogen, methyl, alkoxycarbonyl having 1 to 4 C atoms in the alkoxy moiety or C₁-C₃-acyl,
R¹¹ represents hydrogen or methyl,
R⁸ represents hydrogen, straight-chain or branched C₁-C₃-alkyl or cyclopropyl,
R⁶ represents hydrogen,
R⁹ represents hydrogen or methyl,
R⁵ represents hydrogen or methyl, and
B represents -CH₂-, O or a direct bond.

5. Medicaments comprising compounds of the formula (I) according to Claim 1.

6. Use of compounds of the formula (I) according to Claim 1 for the preparation of medicaments.

7. Use of compounds of the formula (I) according to Claim 1 in antibacterial compositions.

## Revendications

1. Composés répondant à la formule générale (I) 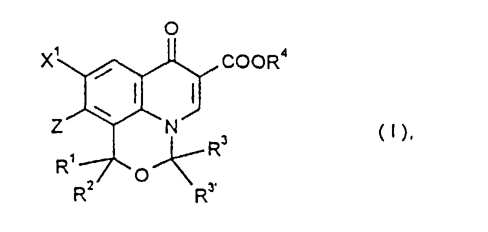 dans laquelle
R¹ représente un atome d'hydrogène ou un groupe alkyle en C₁-C₄ portant le cas échéant un ou plusieurs substituants identiques ou différents hydroxyle ou halogéno,
R² représente, indépendamment de R¹, un atome d'hydrogène ou un groupe méthyle,
R³ représente un atome d'hydrogène ou un groupe alkyle en C₁-C₄,
R^{3'} représente, indépendamment de R³, un atome d'hydrogène ou un groupe méthyle,
R⁴ représente un atome d'hydrogène, un groupe alkyle contenant de 1 à 4 atomes de carbone portant le cas échéant un ou plusieurs substituants identiques ou différents hydroxyle, méthoxy, amino, méthylamino ou diméthylamino, ou encore un groupe (5-méthyl-2-oxo-1,3-dioxol-4-yl)méthyle,
X¹ représente un atome d'hydrogène ou un atome d'halogène,
Z représente des radicaux répondant aux structures 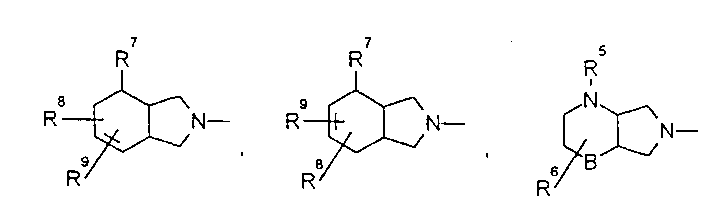 dans lesquelles
R⁷ représente un atome d'hydrogène, un groupe hydroxyle, un groupe -NR¹⁰R¹¹, un groupe hydroxyméthyle ou un groupe -CH₂₋NR¹⁰R¹¹, un groupe carboxyle, un groupe méthoxycarbonyle ou un groupe éthoxy-carbonyle,
dans lesquels
R¹⁰ représente un atome d'hydrogène, un groupe alkyle en C₁-C₃ portant le cas échéant un ou plusieurs substituants hydroxyle, un groupe alcoxycarbonyle contenant de 1 à 4 atomes de carbone dans la fraction alcoxy ou un groupe acyle en C₁-C₃,
R¹¹ représente un atome d'hydrogène ou un groupe méthyle,
R⁸ représente un atome d'hydrogène, un groupe alkyle en C₁-C₃ à chaîne droite ou ramifiée ou un groupe cyclopropyle,
R⁹ représente un atome d'hydrogène ou un groupe méthyle,
R⁶ représente un atome d'hydrogène ou un groupe méthyle,
R⁵ représente un atome d'hydrogène, un groupe méthyle ou des radicaux répondant aux structures -CH=CH-CO₂R^{5'}, -CH₂-CH₂-CO₂R^{5'}, -CH₂-CO-CH₃, -CH₂₋CH₂₋CN,
R^{5'} représente un groupe méthyle ou un groupe éthyle,
B représente un groupe -CH₂, un atome d'oxygène ou une liaison directe.
Les composés répondant à la formule (I) peuvent être présents sous forme de racémates ou comme composés sous forme d'énantiomères purs, ainsi que sous forme de leurs hydrates et de leurs sels d'addition d'acides pharmaceutiquement acceptables, de même que sous forme de leurs sels de métaux alcalins, de métaux alcalino-terreux, d'argent et de guanidinium.

2. Procédé pour la préparation des composés répondant à la formule (I) selon la revendication 1 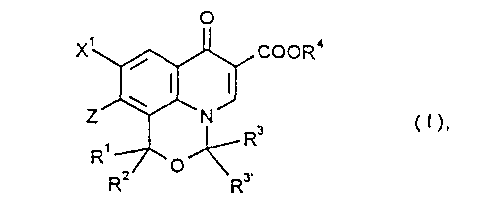 caractérisé en ce qu'on fait réagir des composés répondant à la formule (II) 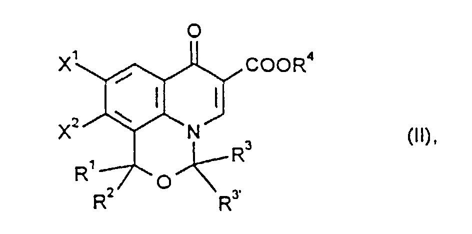 dans laquelle
R¹, R², R³, R^{3'}, R⁴ et X¹ ont la signification indiquée ci-dessus et
X² représente un atome d'halogène, en particulier un atome de fluor ou un atome de chlore,
avec des composés répondant à la formule (III)
Z-H (III)
dans laquelle
Z a la signification indiquée ci-dessus,
le cas échéant en présence d'agents neutralisant les acides.

3. Composés répondant à la formule (I) selon la revendication 1, dans laquelle
R¹ représente un atome d'hydrogène ou un groupe alkyle en C₁-C₃ portant le cas échéant un ou plusieurs substituants hydroxyle,
R² représente, indépendamment de R¹, un atome d'hydrogène ou un groupe méthyle,
R³ représente un atome d'hydrogène, un groupe méthyle ou un groupe éthyle,
R^{3'} représente un atome d'hydrogène ou un groupe méthyle,
R⁴ représente un atome d'hydrogène, un groupe alkyle contenant de 1 à 4 atomes de carbone portant le cas échéant un ou plusieurs substituants identiques ou différents hydroxyle, méthoxy, amino, méthylamino ou diméthylamino, ou encore un groupe (5-méthyl-2-oxo-1,3-dioxol-4-yl)méthyle,
X¹ représente un atome d'hydrogène, un atome de fluor ou un atome de chlore,
Z représente des radicaux répondant aux structures 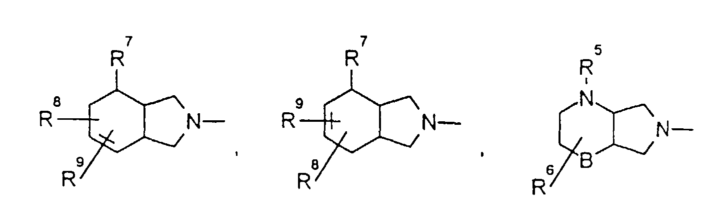 dans lesquelles
R⁷ représente un atome d'hydrogène, un groupe hydroxyle, un groupe -NR¹⁰R¹¹, un groupe hydroxyméthyle ou un groupe -CH₂-NR¹⁰R¹¹,
dans lesquels
R¹⁰ représente un atome d'hydrogène, un groupe alkyle en C₁-C₂ portant le cas échéant un ou plusieurs substituants hydroxyle, un groupe alcoxycarbonyle contenant de 1 à 4 atomes de carbone dans la fraction alcoxy ou un groupe acyle en C₁-C₃,
R¹¹ représente un atome d'hydrogène ou un groupe méthyle,
R⁸ représente un atome d'hydrogène, un groupe alkyle en C₁-C₃ à chaîne droite ou ramifiée ou un groupe cyclopropyle,
R⁹ représente un atome d'hydrogène ou un groupe méthyle,
R⁵ représente un atome d'hydrogène ou un groupe méthyle,
R⁶ représente un atome d'hydrogène,
B représente un groupe -CH₂, un atome d'oxygène ou une liaison directe.

4. Composés répondant à la formule (I) selon la revendication 1, dans laquelle
R¹ représente un atome d'hydrogène ou un groupe méthyle,
R² représente un atome d'hydrogène,
R³ représente un groupe méthyle ou un groupe éthyle,
R^{3'} représente un atome d'hydrogène ou un groupe méthyle,
R⁴ représente un atome d'hydrogène, un groupe méthyle ou un groupe éthyle,
X¹ représente un atome de fluor,
Z représente des radicaux répondant aux structures 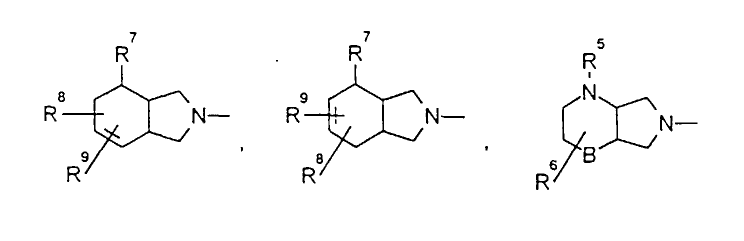 dans lesquelles
R⁷ représente un atome d'hydrogène, un groupe hydroxyle, un groupe -NR¹⁰R¹¹, un groupe hydroxyméthyle ou un groupe -CH₂-NR¹⁰R¹¹,
dans lesquels
R¹⁰ représente un atome d'hydrogène, un groupe méthyle, un groupe alcoxycarbonyle contenant de 1 à 4 atomes de carbone dans la fraction alcoxy ou un groupe acyle en C₁-C₃,
R¹¹ représente un atome d'hydrogène ou un groupe méthyle,
R⁸ représente un atome d'hydrogène, un groupe alkyle en C₁-C₃ à chaîne droite ou ramifiée ou un groupe cyclopropyle,
R⁶ représente un atome d'hydrogène,
R⁹ représente un atome d'hydrogène ou un groupe méthyle,
R⁵ représente un atome d'hydrogène ou un groupe méthyle,
B représente un groupe -CH₂, un atome d'oxygène ou une liaison directe.

5. Médicaments contenant des composés répondant à la formule (I) selon la revendication 1.

6. Utilisation de composés répondant à la formule (I) selon la revendication 1 pour la préparation de médicaments.

7. Utilisation de composés répondant à la formule (I) selon la revendication 1 dans des agents antibactériens.
